# EUROPÄISCHE PATENTSCHRIFT

(11) **EP 3 964 617 B1**
(45) Veröffentlichungstag und Bekanntmachung des Hinweises auf die Patenterteilung: **09.10.2024**
(21) Anmeldenummer: 21186991.2
(22) Anmeldetag: 21.07.2021
(51) Int. Cl.: D04C 1/06, D04C 3/32, D04C 3/48

(54) **COMPUTER-GESTÜTZTES VERFAHREN ZUM ERSTELLEN EINES FLECHTPROGRAMMS, COMPUTERPROGRAMM ZUM ERSTELLEN EINES FLECHTPROGRAMMS UND VORRICHTUNG ZUM ERSTELLEN EINES FLECHTPROGRAMMS**
COMPUTER-AIDED METHOD FOR CREATING A BRAIDING PROGRAM, COMPUTER PROGRAM FOR CREATING A BRAIDING PROGRAM AND A DEVICE FOR CREATING A BRAIDING PROGRAM
PROCÉDÉ ASSISTE PAR ORDINATEUR DE CRÉATION D'UN PROGRAMME DE TRESSAGE, PROGRAMME INFORMATIQUE DE CRÉATION D'UN PROGRAMME DE TRESSAGE ET DISPOSITIF DE CRÉATION D'UN PROGRAMME DE TRESSAGE

(30) Priorität: 03.09.2020 DE 102020005407
(43) Veröffentlichungstag der Anmeldung: 09.03.2022
(73) Patentinhaber: ADMEDES GmbH, 75179 Pforzheim (DE)
(72) Erfinder: BUDILLON, Florent, Pforzheim (DE); LEHMANN, Kevin, Pforzheim (DE); BRAEUNER, Marc O., Pforzheim (DE)
(74) Vertreter: Müller-Boré & Partner Patentanwälte PartG mbB

(56) Entgegenhaltungen:
- EP-A1- 2 977 496
- WO-A1-2019/158760
- DE-A1- 102017 127 943

## Beschreibung

Die Erfindung betrifft ein computer-gestütztes Verfahren zum Erstellen eines Flechtprogramms, ein Computerprogramm zum Erstellen eines Flechtprogramms und eine Vorrichtung zum Erstellen eines Flechtprogramms, wobei das Flechtprogramm maschinenlesbare Befehle umfasst, die bei Ausführen des Flechtprogramms eine Flechtmaschine veranlassen, ein Geflecht, insbesondere ein zumindest teilweise geflochtenes Implantat, herzustellen.

Zum Herstellen eines Geflechts, insbesondere eines medizinischen Implantats (z.B. eines Stents) wird herkömmlicherweise ein Gegenstand mit einem Flechtmaterial umflochten. Das Flechtmaterial wird dabei von einem oder mehreren Klöppeln abgewickelt, wobei die Klöppel um den zu umflechtenden Gegenstand herum geführt werden - meist entlang sich periodisch kreuzenden Laufbahnen. Der zu umflechtende Gegenstand (auch Flechtdorn genannt, z.B. ein im Wesentlichen zylindrischer Körper), wird meist relativ zu den Klöppeln und parallel zu der Rotationsachse der Klöppellaufbahnen verlagert. Je nach Bewegung des Flechtdorns und/oder der Klöppel können eine Vielzahl von Geflechten mit unterschiedlichen Flechtmustern und/oder Flechtgeometrien erzeugt werden. Es sind Flechtmaschinen bekannt, die es ermöglichen, beliebige Flechtmuster zu erzeugen. Dabei werden verschiedene Laufbahnen mittels mechanischer Weiche miteinander verbunden bzw. kombiniert, um Klöppel auf verschiedenen Laufbahnen zu führen und dadurch unterschiedliche Flechtmuster zu erreichen.

WO 2019/158760 A1 beschreibt einen Stent sowie ein Verfahren zum Flechten eines Stents mit einem primären Schenkel und mindestens zwei sekundären Schenkeln. EP 2 977 496 A1 beschreibt ein Verfahren zum Herstellen eines Körperimplantats mit flexiblen Formen. DE 10 2017 127943 A1 beschreibt eine Steuereinrichtung für eine Flechtanlage.

Um Geflechte mit komplexen Formen und/oder Flechtmustern herzustellen, bedarf es einer aufwändigen und exakten Abstimmung der Klöppelbewegungen und der Verlagerung des Flechtdorns. Auch die Beschaffenheit (z.B. Material und Oberfläche) des Flechtdorns und des Flechtmaterials müssen insbesondere berücksichtigt werden. Dies verursacht einen hohen Aufwand für das Erstellen eines entsprechenden Flechtprogramms, um eine Flechtmaschine derart zu steuern, dass ein Geflecht mit den gewünschten Parametern entsteht. Auch werden oftmals viele Testreihen durchgeführt und die Steuerung der Flechtmaschine wird aufwändig manuell angepasst. Ein solcher Vorgang ist sehr zeit- und kostenintensiv und ist insbesondere bei Sonderanfertigungen bzw. Kleinserien kaum wirtschaftlich rentabel. Daneben kann sich die Beschaffenheit des Flechtdorns zwischen Flechtvorgängen ändern, sodass ebenfalls eine Anpassung der Steuerung der Flechtmaschine erforderlich werden kann.

Es ist daher eine Aufgabe der vorliegenden Erfindung, ein computer-gestütztes Verfahren zum Erstellen eines Flechtprogramms bereitzustellen. Insbesondere ist es eine Aufgabe der vorliegenden Erfindung, ein Verfahren zum Erstellen eines Flechtprogramms bereitzustellen, welches einen flexiblen und kostengünstigen Einsatz ermöglicht.

Diese Aufgabe wird durch die Gegenstände der unabhängigen Ansprüche gelöst. Vorteilhafte Ausführungsformen sind Gegenstand der Unteransprüche.

Ein erster Aspekt der Erfindung betrifft ein computer-gestütztes Verfahren zum Erstellen eines Flechtprogramms, wobei das Flechtprogramm maschinenlesbare Befehle umfasst, die beim Ausführen des Flechtprogramms eine Flechtmaschine veranlassen, ein Geflecht, insbesondere ein zumindest teilweise geflochtenes Implantat, herzustellen, wobei das Verfahren umfasst: a) Erzeugen eines Geflechts durch die Flechtmaschine, insbesondere durch wenigstens teilweises Umflechten zumindest eines Flechtdorns und/oder zumindest eines Stehfadens/Stehdrahts, mit einem Flechtmaterial gemäß einer vorgegebenen Konfiguration von Befehlen; b) automatisiertes Erfassen zumindest einer Kenngröße des Geflechts; c) automatisiertes Abgleichen der erfassten Kenngröße des Geflechts mit zumindest einer zugehörigen Soll-Kenngröße; sofern die erfasste Kenngröße der zugehörigen Soll-Kenngröße nicht entspricht: c1-a) Entflechten zumindest eines Bereichs des Geflechts durch die Flechtmaschine; und c1-b) automatisiertes Ändern zumindest eines Teils der Konfiguration von Befehlen zum Erzeugen des Geflechts sowie Wiederholen der Schritte a) bis c) auf der Grundlage der geänderten Konfiguration von Befehlen; sofern die erfasste Kenngröße der zugehörigen Soll-Kenngröße entspricht: c2) Erstellen eines Flechtprogramms gemäß der Konfiguration von Befehlen bei der die erfasste Kenngröße der zugehörigen Soll-Kenngröße entspricht.

Somit wird ein automatisiertes Überprüfen des erzeugten Geflechts ermöglicht und ein automatisches Erstellen bzw. Anpassen der Steuerung des Flechtvorgangs ermöglicht. Außerdem kann auf diese Weise ein Ausschuss von fehlerhaften Geflechten vermieden, zumindest aber deutlich reduziert werden, da ein Geflecht mit einer detektierten Kenngrößenabweichung noch während des Herstellungsverfahrens korrigiert wird.

Bevorzugt ist das Ändern zumindest eines Teils der Konfiguration von Befehlen abhängig von: Art der abweichenden Kenngröße; Ausmaß der Abweichung der Kenngröße; Anzahl der Klöppel, Art des Flechtmaterials; Dicke des Flechtmaterials; Oberflächenbeschaffenheit des Flechtmaterials; Oberflächenbeschaffenheit eines Flechtdorns; und/oder Geometrie eines Flechtdorns.

Ein oder mehrere der genannten, beispielhaften Größen bzw. Werte sind bevorzugt bei der Änderung der Konfiguration von Befehlen zu berücksichtigen da insbesondere diese Größen bzw. Werte einen zumindest geringfügigen, feststellbaren Einfluss auf das zu erzeugende Geflecht ausüben. Beispielsweise erfordern verschiedene Flechtmaterialien, d.h. Flechtmaterial unterschiedlichen Materials, unterschiedlicher Dicke (Durchmesser), etc., eine entsprechend angepasste Änderung der Konfiguration von Befehlen, um eine bestimmte Anpassung des Flechtmusters des damit erstellten Geflechts zu erreichen. Zum Beispiel wirkt sich eine Beaufschlagung einer bestimmten Spannkraft (Wert, mit der das Flechtmaterial während des Flechtvorgangs auf Spannung gehalten wird) auf ein Flechtmaterial mit einem dünneren Durchmesser stärker aus als eine Beaufschlagung derselben Spannkraft auf ein Flechtmaterial mit einem dickeren Durchmesser. Anders ausgedrückt: Um eine bestimmte Änderung des Flechtmusters, welches sich insbesondere aus der Flechtbindung, dem Flechtwinkel und/oder dem Flechtprofil zusammensetzt, eines Geflechts zu erwirken, könnte bei einem dünneren Flechtmaterial eine geringere Änderung einer Größe bzw. eines Werts (beispielsweise der Spannkraft) ausreichen, wobei bei einem dickeren Flechtmaterial eine größere Änderung erforderlich wäre. Ähnliches gilt entsprechend für andere das Flechtmuster eines Geflechts beeinflussende Größen und Werte, welche hier nicht explizit aufgeführt sind.

Gemäß einer besonders bevorzugten Ausführungsform wird das Ändern der Konfiguration von Befehlen durch einen lernfähigen, computerimplementierten Algorithmus beeinflusst. Beispiele hierfür sind maschinelles Lernen und künstliche Intelligenz, insbesondere überwachtes Lernen, sowie Programme, welche zu (teil)autonomen Verhalten und Bewerten von bestimmten Aktionen fähig sind.

Insbesondere bei großen Mengen von Daten wird die Fähigkeit des Menschen, diese Daten aufzunehmen, zu interpretieren und auf ihrer Basis komplexe Entscheidungen zu treffen, überschritten. Künstliche Intelligenz und/oder maschinelles Lernen bildet die Grundlage für lernende Computersysteme und ist in der Lage komplexe Entscheidungsprozesse durchzuführen. Vor allem bei einem großen Umfang an möglichen Kombinationen und/oder Permutationen von Parametern, Werten und/oder Variablen können solche Systeme und Algorithmen beim Finden einer optimalen, zumindest aber vorteilhaften, und/oder schnelleren Lösung eingesetzt werden. So auch bei einer bevorzugten Ausführungsform der vorliegenden Erfindung, da unter anderem eine große Anzahl an Parametern vorliegt, welche das fertige Geflecht beeinflussen. Ebenso besteht eine Fülle von Änderungs- und Kombinationsmöglichkeiten eines oder mehrerer dieser Parameter.

Im Vergleich zu einem auf künstlicher Intelligenz/maschinellem Lernen basierendem Algorithmus führt beispielsweise ein iteratives und/oder zufälliges Ändern einer oder mehrerer Parameter häufig nur mühsam oder gar nicht zu einer zufriedenstellenden Lösung. Durch den Einsatz von künstlicher Intelligenz/maschinellem Lernen ist es vorteilhafterweise möglich, schneller zu einer Lösung zu gelangen und/oder eine überlegene Lösung zu erhalten.

Darüber hinaus können bei der Herstellung eines Geflechts die Auswirkungen von Parameteränderungen nur schlecht oder gar nicht computergestützt simuliert werden, sodass Auswirkungen von Parameteränderungen auf das Flechtergebnis häufig nur durch einen entsprechend durchgeführten Flechtvorgang festgestellt werden kann. Dies kann jedoch zu einem erheblichen Mehraufwand führen. Mithilfe von künstlicher Intelligenz/maschinellem Lernen kann häufig die Anzahl an benötigten Flechtvorgängen zur Findung einer optimalen, zumindest aber vorteilhaften, Lösung reduziert werden.

Beispielsweise kann der lernfähige, computerimplementierte Algorithmus die Konfiguration von Befehlen ändern auf Basis: einer vorgegebenen Konfiguration von Befehlen; einer zuvor erfassten Kenngrößenabweichung; einer durchgeführten Konfigurationsänderung; und/oder einer detektierten Auswirkung einer bestimmten Konfigurationsänderung.

Bevorzugt ist der lernfähige, computerimplementierte Algorithmus eingerichtet, auf Informationen zuzugreifen und diese bei der automatisierten Änderung der Konfiguration von Befehlen zu berücksichtigen. Besonders bevorzugt ist dabei ein Algorithmus, der Zugriff auf vorgegebene Konfigurationen von Befehlen zum Erzeugen eines Geflechts und/oder auf ein oder mehrere bereits erstellte Flechtprogramme hat. Alternativ und/oder zusätzlich ist es von Vorteil, dass der Algorithmus in der Lage ist, auf Informationen betreffend ein oder mehrerer vorgegebener und/oder erfasster Kenngrößenabweichungen und/oder, weiter bevorzugt, ein oder mehrerer vorgegebener und/oder durchgeführter Konfigurationsänderungen zuzugreifen. Vorteilhafterweise kann der Algorithmus dadurch schneller zu einer Lösung gelangen bzw. eine verbesserte/bevorzugte Lösung finden.

Weiter bevorzugt umfasst die Konfiguration von Befehlen zum Erzeugen des Geflechts ein oder mehrere der folgenden Parameter: Verlagerung zumindest eines Klöppels; Verlagerung des Geflechts; Verlagerung eines Flechtdorns; Spannung des Flechtmaterials, Position und/oder Verlagerung zumindest eines Flechtrings und/oder eines Flechtauges.

Wie oben beschrieben wird ein Flechtvorgang insbesondere durch die Vorgabe eines oder mehrerer der aufgeführten, beispielhaften Parameter definiert bzw. gesteuert. Die Konfiguration von Befehlen zum Erzeugen des Geflechts umfasst bevorzugt eine zeitlich und/oder räumlich abgestimmte Abfolge von Befehlen, welche zumindest eine oder mehrere der genannten Parameter steuern.

Vorteilhaftweise beinhaltet eine Kenngröße des Geflechts: Position zumindest einer Überkreuzung des Flechtmaterials (beispielsweise in Relation zu einem oder mehreren anderen Überkreuzungen und/oder zumindest einem Merkmal eines Flechtdorns und/oder einem oder mehreren beliebigen anderen Bezugspunkten, welche nicht Bestandteil des Geflechts sind); Durchmesser des Flechtmaterials; Länge einer Raute; Breite einer Raute; Länge eines Rautensegments; und/oder Flechtwinkel einer Raute. Weitere beispielhafte Kenngrößen sind Rautenfläche, Rautendichte und Geflechtdurchmesser.

Ein Abgleichen einer Position einer Überkreuzung des Geflechts in Relation zu einem oder mehreren (Profil-) Merkmalen eines Flechtdorns ist insbesondere vorteilhaft bei einem profilierten Flechtdorn (z.B. Flechtdorn mit ein oder mehreren Änderungen des Dorndurchmessers), da so speziell Kontenpunkte/Überkreuzungen im Bereich einer Änderung des Dornprofils exakt bzw. mit einer möglichst geringen Abweichung erzeugt und/oder platziert werden können.

Das Erfassen einer Kenngröße des Geflechts erfolgt bevorzugt in zumindest einem bestimmten Bereich des Geflechts, bevorzugt in Relation zu einem Flechtdorn, insbesondere in einem Luftbereich, in einem Ablagebereich und/oder in einem Fixbereich des Geflechts.

Bevorzugt umfasst das Entflechten ein im Wesentlichen umgekehrtes Verlagern zumindest eines Klöppels und/oder des zumindest einen Flechtdorns. Besonders bevorzugt ist der zumindest eine Klöppel derart ausgestaltet, um sowohl ein Erzeugen eines Geflechts als auch ein Entflechten zumindest eines Teils eines Geflechts zu ermöglichen, beispielsweise durch Rückführen des Flechtmaterials in einen Materialspeicher. Eine besonders bevorzugte Ausgestaltung eines solchen Klöppels ist in der Patentschrift DE 10 2011 118 108 B3 beschrieben.

So ist ein vorteilhaftes Entflechten möglich, da keine zusätzlichen (über einen entsprechend ausgestalteten Klöppel hinausgehende) Mittel zum Entflechten bereitgestellt werden müssen.

Weiter bevorzugt erfolgt das Entflechten zumindest bis zu dem Bereich des Geflechts, welcher die von der zugehörigen Soll-Kenngröße abweichende Kenngröße aufweist.

Ein zweiter Aspekt der Erfindung betrifft ein Computerprogramm, umfassend Befehle, die beim Ausführen des Programms durch eine Vorrichtung diese veranlassen, das Verfahren zum Erstellen eines Flechtprogramms gemäß des ersten Aspekts der Erfindung auszuführen.

Ein dritter Aspekt der Erfindung betrifft eine Vorrichtung zum Erstellen eines Flechtprogramms, wobei das Flechtprogramm maschinenlesbare Befehle umfasst, die beim bei- Ausführen des Flechtprogramms eine Flechtmaschine veranlassen, ein Geflecht, insbesondere ein zumindest teilweise geflochtenes Implantat, herzustellen, wobei die Vorrichtung zur Durchführung des Verfahrens gemäß des ersten Aspekts der Erfindung geeignet ist und umfasst: zumindest einen Klöppel zum Führen eines Flechtmaterials, wobei der zumindest eine Klöppel verlagerbar ist, um sich entlang von Laufbahnen, insbesondere von beliebig vorgebbaren Laufbahnen, zu bewegen; eine Verlagerungseinrichtung zum Verlagern des zumindest einen Klöppels entlang einer Laufbahn; einen Abzug zum Verlagern des Geflechts, insbesondere zum Aufnehmen und Verlagern zumindest eines Flechtdorns; eine Steuereinheit zum Steuern des Verlagerns des zumindest einen Klöppels und des zumindest einen Flechtdorns, um so ein Geflecht durch Flechten, insbesondere durch wenigstens teilweises Umflechten des zumindest einen Flechtdorns, mit dem Flechtmaterial zu erzeugen; und ein Prüfmittel zum Erfassen zumindest einer Kenngröße eines von der Vorrichtung erzeugten Geflechts; wobei die Steuereinheit ferner eingerichtet ist zum: a) Erzeugen eines Geflechts durch die Flechtmaschine, insbesondere durch wenigstens teilweises Umflechten zumindest eines Flechtdorns, mit einem Flechtmaterial gemäß einer vorgegebenen Konfiguration von Befehlen; b) automatisiertes Erfassen zumindest einer Kenngröße des Geflechts; c) automatisiertes Abgleichen der zumindest einen erfassten Kenngröße des Geflechts mit zumindest einer zugehörigen Soll-Kenngröße; sofern die erfasste Kenngröße der zugehörigen Soll-Kenngröße nicht entspricht: c1-a) Entflechten zumindest eines Bereichs des Geflechts durch die Flechtmaschine; und c1-b) Ändern zumindest eines Teils der Konfiguration von Befehlen zum Erzeugen des Geflechts sowie Wiederholen der Schritte a) bis c) auf der Grundlage der geänderten Konfiguration von Befehlen; sofern die erfasste Kenngröße der zugehörigen Soll-Kenngröße entspricht: c2) Erstellen eines Flechtprogramms gemäß der Konfiguration von Befehlen bei der die erfasste Kenngröße der zugehörigen Soll-Kenngröße entspricht.

Ein Verlagern des Geflechts durch einen Abzug erfolgt bevorzugt direkt, beispielsweise durch ein räumliches Verschieben des erzeugten Geflechts, und/oder indirekt, beispielsweise durch räumliches Verschieben eines Flechtdorns, um welchen das Geflecht erzeugt wurde.

Im Folgenden werden einzelne Ausführungsformen zur Lösung der Aufgabe anhand der Figuren beispielhaft beschrieben. Dabei weisen die einzelnen beschriebenen Ausführungsformen zum Teil Merkmale auf, die nicht zwingend erforderlich sind, um den beanspruchten Gegenstand auszuführen, die aber in bestimmten Anwendungsfällen gewünschte Eigenschaften bereitstellen. So sollen auch Ausführungsformen als unter die beschriebene technische Lehre fallend offenbart angesehen werden, die nicht alle Merkmale der im Folgenden beschriebenen Ausführungsformen aufweisen. Ferner werden, um unnötige Wiederholungen zu vermeiden, bestimmte Merkmale nur in Bezug auf einzelne der im Folgenden beschriebenen Ausführungsformen erwähnt. Es wird darauf hingewiesen, dass die einzelnen Ausführungsformen daher nicht nur für sich genommen, sondern auch in einer Zusammenschau betrachtet werden sollen. Anhand dieser Zusammenschau wird der Fachmann erkennen, dass einzelne Ausführungsformen auch durch Einbeziehung von einzelnen oder mehreren Merkmalen anderer Ausführungsformen modifiziert werden können. Es wird darauf hingewiesen, dass eine systematische Kombination der einzelnen Ausführungsformen mit einzelnen oder mehreren Merkmalen, die in Bezug auf andere Ausführungsformen beschrieben werden, wünschenswert und sinnvoll sein kann und daher in Erwägung gezogen und auch als von der Beschreibung umfasst angesehen werden soll.

### Kurze Beschreibung der Zeichnungen

- Figur 1: zeigt eine beispielhafte Flechtmaschine zum Herstellen eines Geflechts, wobei die Flechtmaschine zur Durchführung des Verfahrens und der Programme gemäß der Aspekte der vorliegenden Erfindung geeignet ist;
- Figur 2: zeigt eine beispielhafte Einrichtung einer Flechtmaschine zur Herstellung einer Vielzahl von unterschiedlichen Geflechten;
- Figur 3A: zeigt ein von einer Flechtmaschine erzeugtes beispielhaftes Geflecht auf einem Flechtdorn;
- Figur 3B: zeigt Beispiele von Flechtbindungen als Flechtmuster-Parameter eines Geflechts;
- Figur 4A: zeigt beispielhafte Kenngrößen eines Geflechts auf Basis derer ein Abgleichen mit einer zugehörigen Soll-Kenngröße erfolgen kann;
- Figur 4B: zeigt ein Geflecht mit beispielhaften Kenngrößenabweichungen;
- Figuren 5A-5D: zeigen beispielhafte Geflechte, welche mit Verfahren, Programmen und/oder Vorrichtungen gemäß Aspekten der vorliegenden Erfindung herstellbar sind;
- Figur 6: zeigt Schritte eines computergestützten Verfahrens zum Erstellen eines Flechtprogramms gemäß einem Aspekt der vorliegenden Erfindung.

### Detaillierte Beschreibung der Zeichnungen

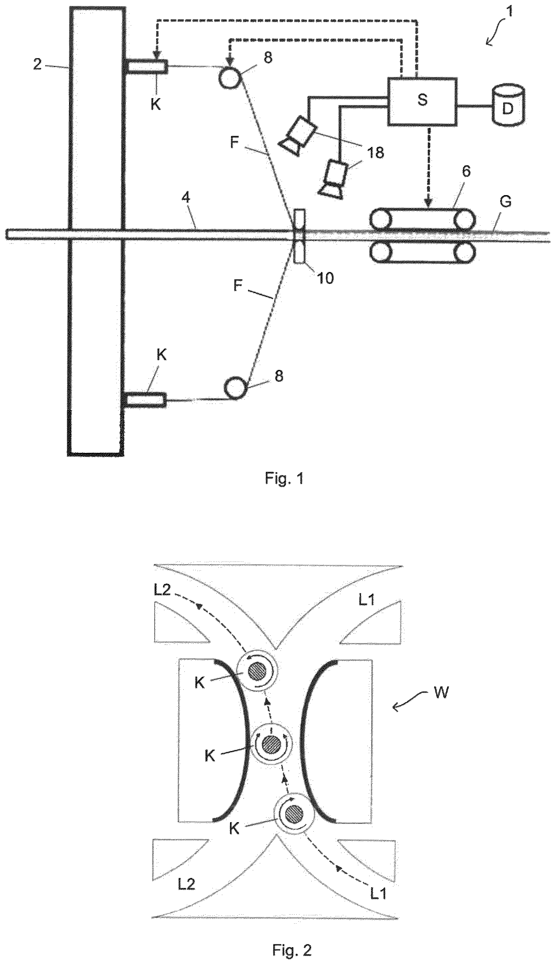

**Figur 1** zeigt eine bespielhafte und bevorzugte Vorrichtung 1 gemäß einer Ausführungsform von einem Aspekt der vorliegenden Erfindung. Die gezeigte Vorrichtung 1 weist eine Verlagerungseinrichtung 2 zum Verlagern eines oder mehrerer (z.B. 2) Klöppel K entlang einer bestimmten Laufbahn auf. Weiter bevorzugt weist die Vorrichtung 1 eine Drehscheibe mit zusätzlichen Flügelrädern auf, auf denen jeweils zumindest ein Klöppel K geführt wird, sodass komplexe und individuelle Laufbahnen der Klöppel K ermöglicht werden.

Optional kann die Vorrichtung 1 einen oder mehrere Flechtringe 8 und/oder ein Flechtauge 10 aufweisen - wie in Figur 1 gezeigt. Ein solcher Flechtring 8 ist bevorzugt zwischen einem Klöppel K und einem Flechtdorn 4 angeordnet, um das aus dem Klöppel K auslaufende Flechtmaterial F zu dem Flechtdorn 4 hin umzulenken. Bevorzugt ist ein Flechtring 8 und/oder ein Flechtauge 10 verlagerbar um einen Winkel des Flechtmaterials F bei Ablegen auf den Flechtdorn 4 zu verändern. Das Flechtmaterial F wird bevorzugt von Spindeln bzw. Spulen der Klöppel K abgezogen. Denkbar ist auch, dass das Flechtmaterial F von einem Materialspeicher (nicht gezeigt) mittels eines Verbindungselementes am Klöppel K befestigt bzw. geführt bzw. gespannt wird, wie beispielsweise in der Patentschrift DE 10 2014 016 381 B4 gezeigt. Zur Steuerung bzw. Aufrechterhaltung der Spannung des Flechtmaterials F kann die Vorrichtung 1 bevorzugt Gewichts- und/oder Federsysteme aufweisen. Vorteilhafterweise kann die Spannung des Flechtmaterials F individuell gesteuert bzw. dynamisch während des Flechtvorgangs verändert bzw. angepasst werden.

Die Vorrichtung 1 ist bevorzugt geeignet zur Herstellung von zumindest teilweise geflochtenen Körperimplantaten bzw. Stents. Diese schützen Kanäle lebender Körper wie beispielsweise Blutgefäße, Speiseröhre, Harnröhre oder Nierengänge durch Einführen des Stents und Expandieren des Stents im Inneren des Körperkanals. Auf diese Weise kann ein Kollabieren oder Verschließen des jeweiligen Körperkanals verhindert werden. Darüber hinaus wird ein Stent beispielsweise für intercerebrale Gefäßaussackungen, sogenannte Aneurysmen eingesetzt, die die häufigste Ursache für nicht-traumatische Subarachnoidal-Blutungen sind. Ihre Inzidenz liegt in der Gesamtbevölkerung bei 1%, nach Autopsiestudien sogar bis zu 9%. Patomorphologisch sind intercerebrale Aneurysmen in der Regel echte, sacurale Aneurysmen, die meist in Gefäßaufzweigungen lokalisiert sind (vgl. beispielsweise Schumacher M. "Diagnostic work-up in cerebral aneurysms" in Nakstadt PHj (ed): "cerebral aneurysms", pp 13-24, Bologna: Centauro (2000)).

Darüber hinaus können derartige Körperimplantate bzw. Stents als Träger von Medikamenten dienen, um eine lokale Therapie innerhalb des Körperkanals zu ermöglichen.

Als Flechtmaterial F wird bei solchen Körperimplantaten vorzugsweise Nitinol verwendet, welches vorzugsweise in einem Salzbad einer Wärmebehandlung unterzogen wurde, um vorgegebene Formgedächtniseigenschaften in das Nitinol einzuprägen. Nitinol ist eine Nickel-Titan-Legierung mit Formgedächtniseigenschaften und hat eine geordnet-kubische Kristallstruktur, die sich von der von Titan und Nickel unterscheidet. Es besteht zum Großteil aus Nickel (ca. 55 %) und einem weiteren großen Anteil Titan. Die Legierung ist korrosionsbeständig, hochfest und superelastisch. Eine typische Anwendung ist wegen der großen Verformbarkeit und der guten Korrosionsfestigkeit chirurgisches Werkzeug, Endoskope oder Implantate, wie beispielsweise Stents. Eine Legierung mit einer hohen Transformationstemperatur von beispielsweise 80 °C wird auch als Memory-Metall oder Formgedächtnismetall bezeichnet. Allerdings können auch eine Vielzahl andere Materialien zur Anwendung kommen, wie beispielsweise Edelstahl.

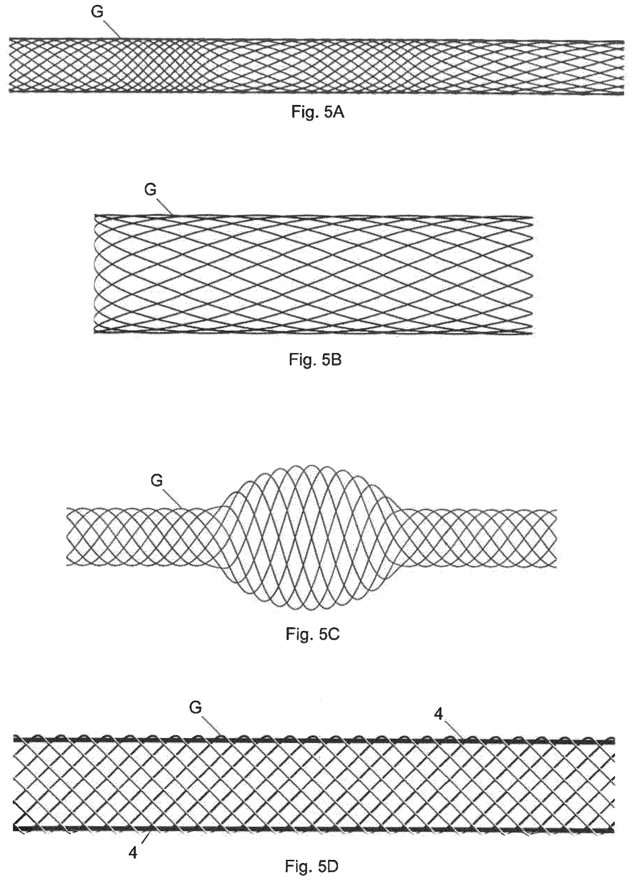

Erfindungsgemäß weist die Vorrichtung 1 einen Abzug 6 zum Verlagern des Geflechts, insbesondere zum Aufnehmen und Verlagern eines Flechtdorns 4, auf. Der Abzug 6 ist vorzugsweise dazu geeignet, das Geflecht und/oder einen Flechtdorn 4 linear, relativ zu den Klöppeln K und/oder parallel zu der Rotationsachse der Klöppellaufbahnen zu verlagern. Durch ein solches relatives Verlagern wird das Geflecht G erzeugt, beispielsweise entlang bzw. an der Oberfläche des Flechtdornkörpers 4. Das Verlagern der ein oder mehreren Klöppel K und des Geflechts G bzw. eines Flechtdorns 4 erfolgt gemäß einer Konfiguration von Befehlen, die von einer Steuereinheit S bereitgestellt werden und welche die Verlagerungseinrichtung 2 und/oder den Abzug 6 entsprechend steuern bzw. regeln. Auf diese Weise wird ein bestimmtes Geflecht G durch Flechten, insbesondere durch zumindest teilweises Umflechten des Flechtdorns 4, mit dem Flechtmaterial F erzeugt. In analoger Weise ist die Vorrichtung 1 geeignet, ein Flachgeflecht zu erzeugen, insbesondere durch Flechten von Flechtmaterial F an bzw. um bzw. entlang wenigstens eines Teils zumindest eines Flechtdorns 4 und/oder eines oder mehrerer Stehfäden/Stehdrähte und Verlagern des Geflechts bzw. des zumindest einen Flechtdorns 4 (siehe beispielsweise Figur 5D).

Weiter bevorzugt ermöglicht es die Vorrichtung 1, beliebige Flechtmuster zu erzeugen. Dabei werden beispielsweise verschiedene Laufbahnen von Klöppeln K mittels mechanischer Weiche W miteinander verbunden bzw. kombiniert, um Klöppel K auf verschiedenen Laufbahnen zu führen und dadurch unterschiedliche Flechtmuster zu erreichen. Ein solcher Vorgang ist in Fig. 2 gezeigt.

Darüber hinaus weist die Vorrichtung 1 zumindest ein Prüfmittel 18 auf, um das Geflecht G zu prüfen. Dabei sind die ein oder mehreren Prüfmittel 18 dazu geeignet, ein oder mehrere Kenngrößen des Geflechts G zu erfassen und/oder mit einer jeweilig zugehörigen Soll-Kenngröße abzugleichen. Auf diese Weise kann die Vorrichtung 1 prüfen, ob eine Anpassung bzw. Änderung der Konfiguration von Befehlen durchgeführt werden soll, gemäß derer das Geflecht G erzeugt wird. Ein solches Prüfmittel 18 kann beispielsweise eine Kamera, ein Mikroskop, einen Laserscanner, einen Messkopf, ein Computer-Tomographie-Gerät und/oder andere Vorrichtungen geeignet zum Erfassen einer oder mehrerer Kenngrößen des Geflechts aufweisen. Insbesondere können mehrere identische und/oder unterschiedliche Prüfmittel 18 bereitgestellt werden. Der Prüfvorgang kann im Wesentlichen kontinuierlich, in konstantem und/oder in variablem Takt erfolgen.

Außerdem weist die Vorrichtung 1 eine Steuereinheit S auf. Die Steuereinheit S ist mit den ein oder mehreren Prüfmitteln 18 signalverbunden und derart eingerichtet, um Informationen über ein oder mehrere der erfassten Kenngrößen des Geflechts G (z.B. via Leitung(en) und/oder kabellos) zu empfangen. Von Vorteil verfügt die Steuereinheit S über einen Zugriff auf eine Datenbank D, wobei die Datenbank D beispielsweise Konfigurationen von Befehlen zum Erzeugen eines Geflechts, ein oder mehrere Flechtprogramme zum Steuern einer Flechtmaschine und/oder Informationen bezüglich bekannter bzw. vorgegebener Kenngrößen bzw. Kenngrößenabweichungen umfasst. Dabei ist die Steuereinheit S derart eingerichtet, dass diese zu einem Abgleich von einer oder mehreren erfassten Kenngrößen des Geflechts G mit zumindest einer vorgebbaren bzw. vorgegebenen Soll-Kenngröße eingerichtet ist. Auch ist die Steuereinheit S zur Steuerung bzw. Regelung der Verlagerung des zumindest einen Klöppels K und/oder des Geflechts G bzw. des zumindest einen Flechtdorns 4 eingerichtet. Bevorzugt kann die Steuereinheit S ein oder mehrere verlagerbare Flechtringe 8 verlagern und/oder Merkmale eines Flechtauges 10 (wie z.B. Innendurchmesser, Einlauf-Radius, Material, Oberflächenbeschaffenheit, Reibungseigenschaft, Position und/oder Ausrichtung) steuern bzw. regeln.

Darüber hinaus ist die Steuereinheit 8 der Vorrichtung 1 geeignet, eine Konfiguration von Befehlen, auf Basis derer ein Geflecht G durch Flechten, insbesondere durch wenigstens teilweises Umflechten zumindest eines Flechtdorns 4, erzeugt wird, zumindest teilweise zu Ändern und, weiter bevorzugt, die Vorrichtung 1 gemäß der der geänderten Konfiguration von Befehlen zu steuern.

**Figur 2** zeigt ein beispielhaftes Verfahren zur komplexen Verlagerung zumindest eines Klöppels K, zu welchem eine besonders bevorzugte Vorrichtung 1 eingerichtet ist. Bei diesem beispielhaften Verfahren erfolgt eine Übergabe bzw. ein Übergabevorgang eines Führungselements eines Klöppels K von der Laufbahn L1 eines rechten Flügelrads (nicht dargestellt) zu der Laufbahn L2 eines linken Flügelrads (nicht dargestellt). Nach dem Eintreten des Führungselements in die Weiche W wird das Führungselement an das linke Flügelrad übergeben. Dies kann beispielsweise durch entsprechend gesteuerte Elektromagnete und/oder mechanische Aktuatoren im Weichenbereich erfolgen. Auf diese Weise wird das Führungselement und somit der Klöppel K von einer Laufbahn L1 auf eine andere Laufbahn L2 überführt. Die Verlagerungseinrichtung 2 der Vorrichtung 1 gemäß Fig. 1 weist bevorzugt eine oder mehrere solcher oder alternativer Einrichtung zum individuellen und anpassbaren Verlagern eines oder mehrerer Klöppel K entlang einer komplexen Laufbahn auf. Eine besonders bevorzugte Ausgestaltung einer Vorrichtung bzw. eines Verfahrens zum Verlagern zumindest eines Klöppels K ist in der Patentschrift DE 10 2016 013 486 B3 beschrieben.

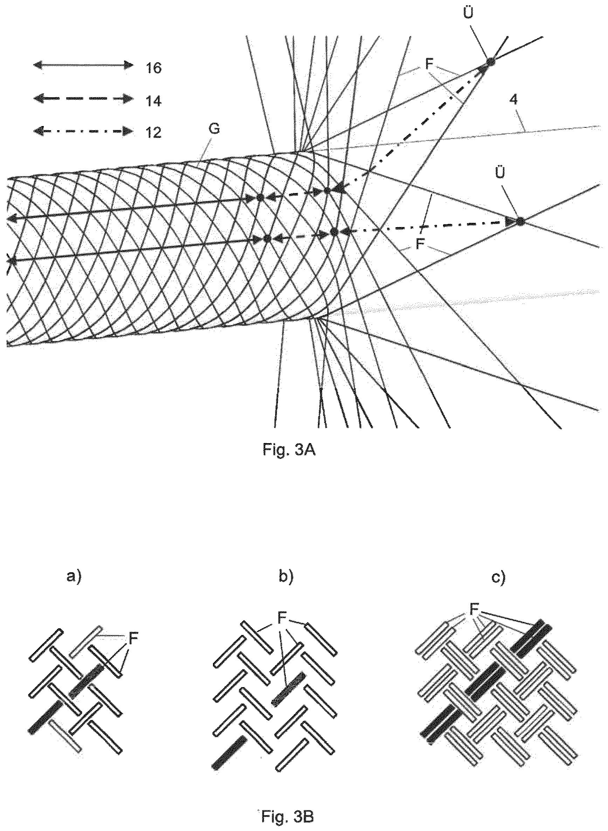

Mit Verweis auf **Figur 3A** sind die ein oder mehreren Prüfmittel 18 der Vorrichtung 1 derart eingerichtet, dass ein oder mehrere Bereiche des Geflechts geprüft werden können. Bevorzugt kann zumindest der Bereich des Geflechts G geprüft werden, in dem das Flechtmaterial F im Wesentlichen fixiert ist bzw. der sich auf einem Flechtdorn 4 befindet und/oder in dem das Flechtmaterial F auf dem Flechtdorn im Wesentlichen fixiert ist (Fixbereich 16). Alternativ und/oder zusätzlich sind die ein oder mehreren Prüfmittel 18 derart eingerichtet, dass es zumindest den Bereich des Geflechts G prüfen kann, in welchem sich das Flechtmaterial F an das Geflecht anlegt bzw. an/auf zumindest einen Stehfaden/Stehdraht bzw. auf den Flechtdorn 4 ablegt (Ablagebereich 14). In dem Ablagebereich 14 des Geflechts G kann sich das Flechtmuster durch ein weiteres Verlagern der Klöppel K und/oder des Flechtdorns 4 weiter verändern. Weiter bevorzugt ist zumindest ein Prüfmittel 18 derart eingerichtet, dass alternativ und/oder zusätzlich zu dem Fixbereich 16 und/oder Ablagebereich 14 der Luftbereich 12 des Geflechts G geprüft wird. Der Luftbereich 12 des Geflechts liegt vor dem Ablagebereich 14 und beginnt beispielsweise an dem Punkt, an dem sich eine Überkreuzung des Flechtmaterials F in der Luft ausbildet.

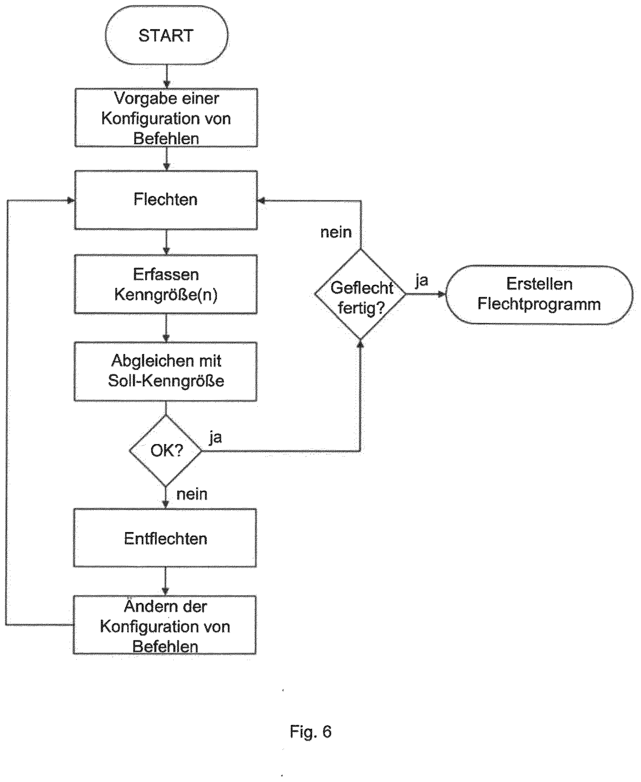

Gemäß einer weiteren, besonders bevorzugten Ausführungsform ist zumindest ein Prüfmittel 18 und die Steuereinheit S derart eingerichtet, dass abweichende Kenngrößen des Geflechts G möglichst frühzeitig erkannt werden, sodass nur ein möglichst kleiner Bereich des Geflechts G zum Berichtigen der abweichenden Kenngröße entflochten werden muss (wie nachfolgend mit Bezug auf **Figur 6** näher beschrieben werden wird).

Darüber hinaus ist es von Vorteil, wenn die Steuereinheit S eingerichtet ist, eine sich ausbildende Kenngrößenabweichung des Geflechts G vorherzusagen und diese gegebenenfalls durch Anpassen der Verlagerung von Klöppel K und/oder Geflecht bzw. Flechtdorn 4 zu verhindern (Echtzeitkorrektur). Alternativ und/oder zusätzlich kann der Flechtvorgang frühzeitig gestoppt werden, beispielsweise noch vor Ablegen des Bereichs des Geflechts G auf den Flechtdorn 4, welcher die abweichende Kenngröße aufweist bzw. aufweisen wird: Dadurch kann eine Verkleinerung des zu entflechtenden Bereichs des Geflechts G und somit eine Verringerung des Korrekturaufwands erreicht werden. Um dies zu erreichen ist es insbesondere vorteilhaft, ein oder mehrere Prüfmittel 18 bereitzustellen. Bevorzugt prüfen die ein oder mehreren Prüfmittel 18 unterschiedliche und/oder sich überlappende Bereiche des Geflechts G bzw. des Flechtmaterials F, wie bezüglich der Figur 3A beschrieben. Auch können bevorzugt verschiedene Prüfmittel 18 vorgesehen sein, um jeweils verschiedene Kenngrößen des Geflechts G bzw. des Flechtmaterials F zu erfassen. Auch kann zumindest ein Prüfmittel 18 vorgesehen sein, um eine Überkreuzung bzw. ein Knotenpunkt Ü des Flechtmaterials F (vgl. Figur 3A) vor Ablegen des Flechtmaterials F auf den Flechtdorn 4 zu detektieren.

Erfindungsgemäß ist die Steuereinheit S der Vorrichtung 1 ferner eingerichtet, um einen Abgleich zwischen einer von einem Prüfmittel 18 erfassten Kenngröße des Geflechts und einer zugehörigen, vorgegebenen bzw. vorgebbaren (Soll-)Kenngröße durchzuführen. Die Soll-Kenngröße wird beispielsweise durch das vorgegebene bzw. gewünschte Flechtmuster des herzustellenden Geflechts G vorgegeben bzw. definiert. Das Flechtmuster wird insbesondere definiert durch ein oder mehrere folgender Flechtmuster-Parameter: Anzahl und/oder Durchmesser -des Flechtmaterials F), Flechtbindung (z.B. 1:1-1, 1:2-1, 1:1-2, ...), Geflechtdurchmesser 32, Geflechtprofil, Länge des Geflechts bzw. bestimmte Bereiche des Geflechts, Flechtwinkel (Winkel zwischen Flechtmaterialien F bzw. Flechtmaterial F und Längsachse des Flechtdorns 4), Flechtdichte (Anzahl an durch das Flechtmaterial gebildeter geometrischer Formen (z.B. Rauten) in einem bestimmten Teilabschnitt bzw. Bereich des Geflechts).

In **Figur 3B** sind beispielhafte Flechtbindungen abgebildet, welche insbesondere ein Flechtmuster eines Geflechts G charakterisieren (Flechtmuster-Parameter). Um verschiedene Geflechte unterscheiden und klassifizieren zu können, ist ein entsprechendes Notationssystem vorteilhaft. In Figur 3B sind beispielhafte - insbesondere in Deutschland bekannte - Notationen von Flechtbindungen abgebildet. Bevorzugt weist die Bezeichnung der Flechtbindung zwei Bestandteile auf: Beispielsweise beschreibt der erste Teil die Überkreuzungsform, der zweite Teil beschreibt die Anzahl der verwendeten Flechtfäden. Die gezeigten Flechtbindungen entsprechen bevorzugten bzw. gängigen Flechtmustern: a) Einflechtig einfädig - 1:1-1; b) Zweiflechtig einfädig - 1:2-1; c) Einflechtig zweifädig - 1:1-2. Ein Geflecht G kann eine einheitliche Flechtbindung oder unterschiedliche Flechtbindungen in verschiedenen Bereichen aufweisen. Die Flechtbindung ist gemäß den zu erzielenden bzw. gewünschten Eigenschaften, wie z.B. Formgebung, Durchlässigkeit und/oder Flexibilität, des Geflechts G bzw. Bereichen des Geflechts G wählbar. Je nach Flechtbindung werden unterschiedliche Konfigurationen von Befehlen zum Herstellen eines Geflechts bereitgestellt bzw. verwendet. Die in Figur 3B abgebildeten Flechtbindungen zeigen zur Veranschaulichung einige Flechtbindungen, wobei die Vorrichtung und das Verfahren gemäß der vorliegenden Erfindung geeignet sind, auch weitere, nicht gezeigte Flechtbindungen zu erzeugen, insbesondere auch Flechtbindungen anderer Notationen.

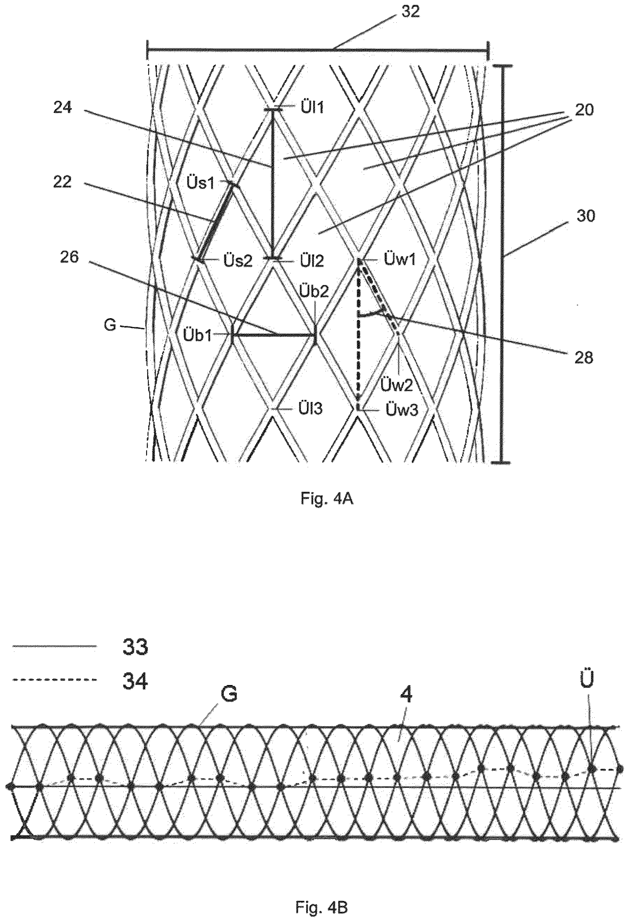

Im Folgenden werden einige der besonders bevorzugten und in **Figur 4A** beispielhaft abgebildeten Kenngrößen bzw. Flechtmusterparameter beispielhaft beschrieben:
Die genannten, beispielhaften Kenngrößen bezeichnen bevorzugte geometrische bzw. strukturelle Merkmale und/oder Eigenschaften eines Geflechts G. Eine Überkreuzung bzw. ein Knotenpunkt Ü des Flechtmaterials F wird beispielsweise durch ein Überkreuzen von zwei Flechtmaterialien bzw. Flechtfäden F von unterschiedlichen Klöppeln gebildet. Mehrere Überkreuzungen bzw. Kontenpunkte Ü (z.B. vier) bilden eine geometrische Form, beispielsweise eine Raute 20.

Ein Rautensegment bzw. ein Rautensteg 22 entspricht bevorzugt einem Abschnitt eines Flechtfadens F, welcher zwei benachbarte Überkreuzungen bzw. Knotenpunkte Ü desselben Flechtfadens F verbindet. In Figur 4A ist dies beispielhaft ein Steg 22 zwischen Kontenpunkten bzw. Überkreuzungen Üs1 und Üs2, Üs2 und Üb1 bzw. Üs1 und Ül2.

Die Länge 24 einer Raute 20 entspricht bevorzugt dem Abstand bzw. der Distanz zwischen zwei Überkreuzungen bzw. Knotenpunkten Ü von jeweils zwei unterschiedlichen Flechtmaterialien bzw. Flechtfäden F, welche im Wesentlichen in axialer Richtung des Dorns 4 bzw. des Geflechts G gegenüberliegend angeordnet sind. Diese Überkreuzungen bzw. Knotenpunkte Ü sind bevorzugt durch zwei Rautensegmente 22 unterschiedlicher Flechtfäden F verbunden. In Figur 4A ist dies beispielhaft der Abstand zwischen Kontenpunkten Ül1 und Ül2, Ül2 und Ül3 bzw. Üw1 und Üw3.

Ähnlich wie die Länge 24 einer Raute 20 entspricht die Breite 26 einer Raute 20 bevorzugt dem Abstand bzw. der Distanz zwischen zwei Überkreuzungen bzw. Knotenpunkten Ü von jeweils zwei unterschiedlichen Flechtfäden F, welche allerdings in im Wesentlichen radialer Richtung des Dorns 4 bzw. des Geflechts G gegenüberliegend angeordnet sind. In Figur 4A ist dies beispielhaft der Abstand zwischen Kontenpunkten Üb1 und Üb2, Üb2 und Üw2 bzw. Ül3 und Üw3.

Der Flechtwinkel 28 einer Raute 20 entspricht bevorzugt dem Winkel eines Rautensegments 22 relativ zu der axialen bzw. zur radialen Richtung des Geflechts G bzw. des Flechtdorns 4. Alternativ kann ein Flechtwinkel 28 den Winkel beschreiben, welcher zwischen zwei Rautensegmenten 22 ausgebildet wird. In Figur 4A ist dies beispielhaft der Winkel zwischen dem Segment 22, welches durch die zwei Kontenpunkte Üw1 und Üw2 gebildet wird, und der axialen Richtung des Flechtdorns 4 bzw. des Geflechts G. Ein weiteres Beispiel ist der Winkel zwischen dem Segment 22, welches durch die zwei Kotenpunkte Üw1 und Üw2 gebildet wird, und dem Segment 22, welches durch die zwei Kontenpunkte Üw2 und Üw3 gebildet wird. Die den Flechtwinkel 28 definierenden Rautensegmente 22 müssen jedoch nicht notwendigerweise einen gemeinsamen Knotenpunkt Ü aufweisen. Beispielsweise kann ein Flechtwinkel 28 auch zwischen gegenüberliegenden Segmenten 22 einer Raute 20 bestimmt werden oder zwischen Segmenten 22 verschiedener Rauten 20.

Die Rautendichte entspricht bevorzugt der Anzahl von Rauten über eine bestimmte Länge, Breite und/oder Fläche zumindest eines Teilabschnitts des Geflechts G. In Figur 4A ist dies beispielhaft die Anzahl der in axialer Richtung aneinander anschließenden Rauten 20 über die Länge des abgebildeten Teilabschnitts 30 des Geflechts G. Bevorzugt wird der entsprechende Teilabschnitt 30 derart gewählt, dass dieser beispielsweise an einem ersten Knotenpunkt Ü einer Raute beginnt und sich entlang einer vorgegebenen Richtung (z.B. eine axiale Richtung des Flechtdorns 4 bzw. des Geflechts G) über eine vorgegebene Länge bzw. Distanz erstreckt. Der Wert der Rautendichte ist beispielsweise die Anzahl von festgelegten Knotenpunkten bzw. Überkreuzungen Ü (z.B. der in Axialrichtung vorderste Kontenpunkt einer Raute ÜL1) in dem festgelegten Teilbereich 30 des Geflechts. Selbiges gilt für eine Fläche und/oder für eine Breite eines Teilabschnitts 30 des Geflechts G.

Die gezeigten bzw. aufgeführten Kenngrößen entsprechen lediglich einer Auswahl von möglichen, geeigneten Kenngrößen des Geflechts G. Die Raute 20 ist eine bei Geflechten, insbesondere zumindest teilweise geflochtenen Implantaten, häufig auftretende erzeugte Form und in der vorliegenden Erfindung lediglich beispielhaft genannt. Es können - insbesondere abhängig von der Anzahl von Flechtfäden F bzw. Klöppeln K und deren Verlagerung - auch andere Formen ausgebildet werden, wodurch zusätzliche und/oder alternative Kenngrößen zum Erfassen und Abgleichen möglich sind.

Bei dem Abgleichen der Kenngröße wird beispielsweise geprüft, ob eine oder mehrere erfasste Kenngrößen einer jeweilig zugehörigen vorgegebenen bzw. vorgebbaren Soll-Kenngröße entsprechen. Besonders bevorzugt ist eine im Wesentlichen exakte Übereinstimmung der erfassten Kenngröße mit der zugehörigen Soll-Kenngröße. Allerdings können Toleranzbereiche existieren, in denen sich eine Abweichung der erfassten Kenngröße von der Soll-Kenngröße bewegen kann, ohne dass das Ergebnis eines Abgleichs negativ ist. Je nach Art der Kenngröße, Messgenauigkeit der Prüfmittel 18 und/oder vorgegebener bzw. gewünschter Fertigungsgenauigkeit können diese Toleranzbereiche variieren (beispielsweise im Bereich von kleiner 20%, bevorzugt kleiner 5%, weiter bevorzugt kleiner 1%). Falls die Abweichung einer Kenngröße innerhalb des definierten Toleranzbereiches liegt, liegt ein positives Ergebnis des Abgleichs vor.

Die in Zusammenhang mit **Figur 1** beschriebene Vorrichtung 1 ist darüber hinaus geeignet, zumindest einen Bereich des erzeugten Geflechts G aufzudröseln bzw. zu entflechten. Damit ein Entflechten erfolgen kann, ist die Steuereinheit S eingerichtet, Klöppel K, Geflecht G, Flechtdorn 4, Weicher W, Flechtringe 8 und/oder Flechtauge 10 entsprechend zu steuern bzw. regeln. Beispielsweise werden dabei ein oder mehrere Klöppel K und/oder das Geflecht G bzw. der Flechtdorn 4 entlang der jeweiligen Laufbahn in umgekehrter Richtung verlagert, sodass ein geordnetes Entflechten zumindest eines Bereichs des Geflechts G erreicht wird. Für den Fall, dass ein negatives Ergebnis eines Abgleichs einer erfassten Kenngröße von einer Soll-Kenngröße vorliegt, d.h. ein Abweichen der Kenngröße außerhalb des definierten Toleranzbereichs, kann so der Flechtvorgang angehalten und bevorzugt zumindest bis zu dem Bereich des Geflechts G entflochten werden, welcher die abweichende Kenngröße aufweist.

Die Steuereinheit S der Vorrichtung 1 ist eingerichtet, die Konfiguration von Befehlen, gemäß derer das Flechten, insbesondere das Umflechten des Flechtdorns 4, durchgeführt wird, zu ändern bzw. anzupassen. Ein oder mehrere Parameter von zumindest einem Teil der Konfiguration von Befehlen können somit durch die Steuereinheit S derart geändert werden, dass ein Abweichen einer erfassten Kenngröße verhindert wird bzw. eine erfasste Kenngröße an eine zugehörige Soll-Kenngröße derart angenähert wird, sodass diese bevorzugt innerhalb eines etwaigen Toleranzbereichs liegt. Die änderbaren Parameter der Konfiguration von Befehlen umfassen beispielsweise diejenigen Parameter, welche verantwortlich sind für die Verlagerung einer oder mehrerer Klöppel K, die Verlagerung des Geflechts G bzw. des Flechtdorns 4 und/oder für das Steuern der Spannung des Flechtmaterials F. Die Verlagerung eines Klöppels K umfasst insbesondere eine räumliche Laufbahn und/oder die Geschwindigkeit mit welcher ein Klöppel K entlang einer Laufbahn bewegt wird. Ebenso kann beispielsweise die Abstimmung bzw. das Timing der Verlagerung von zwei oder mehr Klöppeln K geändert werden, sowie ein Verlagern eines oder mehrerer Flechtringe 8 und/oder Steuern eines Flechtauges 10 und/oder einer Spannung des Flechtmaterials F

Gemäß der Erfindung wird das Ändern zumindest eines Teils der Konfiguration von Befehlen automatisiert durchgeführt. Hierfür eignen sich insbesondere einfache iterative Verfahren oder Optimierungsalgorithmen wie z.B. Gauß-Newton-Verfahren und/oder Gradientenverfahren. Allerdings führt ein solches Vorgehen oftmals zu einer hohen Anzahl an zu evaluierenden Versuchen, wobei bei jedem Misserfolg ein erneutes Entflechten nötig ist. Daher wird gemäß einer besonders bevorzugten Ausführungsform der Erfindung die Änderung der Konfiguration von Befehlen durch einen lernfähigen, computerimplementierten Algorithmus beeinflusst bzw. bestimmt. Durch den Einsatz von künstlicher Intelligenz/maschinellem Lernen ist es vorteilhafterweise möglich, schneller zu einer Lösung zu gelangen und/oder eine überlegene Lösung zu erhalten. Dadurch kann das Finden einer geeigneten Änderung beschleunigt werden, beispielsweise gegenüber den genannten Optimierungsverfahren und randomisierten bzw. willkürlichen Parameteränderungen. Ein solcher Algorithmus ist bevorzugt in der Lage auf Erfahrungen zurückzugreifen, beispielsweise auf bereits detektierte Kenngrößenabweichungen, zur Korrektur durchgeführten Maßnahmen sowie dem Resultat dieser Maßnahmen. Auch Informationen aus der Vorrichtung bekannten, vorgegebenen Konfigurationen von Befehlen können bei dem Finden einer geeigneten Änderung hinzugezogen werden. Auf diese Weise kann ein beschleunigtes Korrigieren der Abweichung durch gezieltes bzw. erfolgversprechendes Ändern der das Umflechten steuernden Konfiguration von Befehlen erreicht werden.

Vorteilhafterweise ist das Ändern zumindest eines Teils der Konfiguration von Befehlen abhängig von beispielsweise der Art der abweichenden Kenngröße, dem Ausmaß der Abweichung der Kenngröße, der Art des Flechtmaterials F, der Dicke des Flechtmaterials F, der Oberflächenbeschaffenheit des Flechtmaterials F, der Oberflächenbeschaffenheit des Flechtdorns 4 und/oder der Geometrie des Flechtdorns 4. Insbesondere diese und ähnliche Eigenschaften bzw. Parameter beeinflussen das Flechtergebnis.

Beispielsweise für den Fall, dass die von einem oder mehreren Prüfmitteln 18 erfasste Kenngröße der Rautenlänge 24 zu gering ist: In diesem Fall kann beispielsweise eine Erhöhung der Verlagerungsgeschwindigkeit des Geflechts G bzw. des Flechtdorns 4 (z.B. durch entsprechendes Steuern bzw. Regeln des Abzugs 6) zur Korrektur dienen. Bei einem zufälligen Ändern der Konfiguration von Befehlen müssen sowohl der oder die zu ändernden Parameter der Konfiguration von Befehlen, als auch der Umfang dieser Parameteränderung durch ein aufwändiges trial-and-error-Verfahren herausgefunden werden. Dies ist in der Regel zeit- und kostenintensiv. Mit dem Verfahren gemäß der beschriebenen, besonders bevorzugten Ausführungsform kann beispielsweise auf Erfahrungen zurückgegriffen werden und eine Parameteränderung vorgenommen werden, die eine möglichst hohe Erfolgsaussicht aufweist. Vorteilhafterweise kann alternativ und/oder zusätzlich eine bekannte Konfiguration von Befehlen, welche ein Erzeugen eines Geflechts mit länger werdenden Rauten umfasst, als Grundlage bzw. unterstützende Information auf der Suche nach einer geeigneten Parameteränderung dienen. Auf diese Weise kann die Zeit und/oder die Anzahl der benötigten Versuche zum Erreichen des gewünschten bzw. vorgegebenen Flechtmusters des Geflechts G verringert werden.

Ebenso kann beispielsweise durch eine stetige Änderung der Oberflächenbeschaffenheit des Flechtdorns 4 durch Abnutzung eine entsprechende Anpassung des Flechtvorgangs vorteilhaft sein. Die Steuereinheit der Vorrichtung 1 kann auf eine in der Vergangenheit durchgeführte und die Abnutzung kompensierende Konfigurationsänderungen zurückgreifen und so schneller eine geeignete Anpassung der Konfiguration von Befehlen erreichen.

Bei einem weiteren beispielhaften Anwendungsfall wird eine Abweichung eines Flechtwinkels 28 des Geflechts G von einem oder mehreren Prüfmitteln 18 (z.B. ein oder mehrere Kameras) erfasst. Eine entsprechende vorteilhafte Änderung der Konfiguration von Befehlen kann beispielsweise ein Ändern des Verlagerns eines oder mehrerer Klöppel K bei gleichzeitigem Beschleunigen oder Verlangsamen des Verlagerns des Geflechts G bzw. des Flechtdorns 4 umfassen. Bei der Entscheidungsfindung bezüglich des zu ändernden Parameters bzw. der zu ändernden Parameter und/oder bei der Entscheidungsfindung bezüglich des Umfangs und Art der Änderung kann die Steuereinheit S bevorzugt auf in einer Datenbank D abgelegte Flechtprogramme, Konfiguration von Befehlen und/oder bereits durchgeführte bzw. vorgegebene Konfigurationsänderungen zugreifen. Beispielsweise kann die Steuereinheit S so auf Informationen bezüglich Klöppelverlagerung und/oder Geflecht- bzw. Flechtdornverlagerung von einem Flechtprogramm zum Flechten eines Geflechts G mit sich kontinuierlich änderndem Flechtwinkel zugreifen und die vorgegebene bzw. aktuelle Konfiguration von Befehlen auf Basis dessen anpassen.

**Figur 4B** zeigt ein Geflecht G mit beispielhaften Kenngrößenabweichungen. Das abgebildete Geflecht G weist Knotenpunkte/Überkreuzungen Ü auf, wobei zumindest deren Position (verdeutlicht durch Verbindungslinie 34) in Relation zu dem Flechtdorn 4 bzw. zu dessen Längsachse 33 abweicht. Die dazugehörigen, vorgegebenen bzw. vorgebbaren Positionen (Soll-Kenngrößen) der Kontenpunkte bzw. Überkreuzungen Ü des Geflechts G befinden sich in diesem Beispiel auf bzw. entlang der Längsachse 33 des Flechtdorns 4. Die gezeigten, beispielhaften Abweichungen der "Geradheit des Geflechts" sind durch Ändern der Konfiguration von Befehlen, insbesondere durch Ändern der Parameter zum Steuern bzw. Regeln eines oder mehrerer Flechtringe 8 und/oder Flechtaugen 10, korrigierbar.

Das Umflechten wird jeweils mit der zuletzt geänderten Konfiguration von Befehlen wiederaufgenommen bzw. gestartet und wird solange fortgesetzt, bis eine weitere Kenngrößenabweichung detektiert wird oder das Geflecht G fertiggestellt wurde. Sollte eine Kenngrößenabweichung detektiert werden, wird eine entsprechende Änderung der Konfiguration von Befehlen durchgeführt und - nach Entflechten des betroffenen Bereichs - das Erzeugen des Geflechts G durch Flechten, insbesondere durch wenigstens teilweises Umflechten des zumindest einen Flechtdorns 4, gemäß der geänderten Konfiguration von Befehlen fortgesetzt. Wenn keine (weitere) Kenngrößenabweichung detektiert wird und das Geflecht fertiggestellt wurde, wird ein Flechtprogramm erstellt, welches die Konfiguration von Befehlen umfasst, bei der alle erfassten Kenngrößen den zugehörigen Soll-Kenngrößen entsprechen. Das erstellte Flechtprogramm umfasst Befehle, welche bei Ausführung des Programms eine Flechtmaschine veranlassen, ein Geflecht G mit den gewünschten Eigenschaften durch Flechten, insbesondere durch wenigstens teilweises Umflechten zumindest eines Flechtdorns 4, mit einem Flechtmaterial F gemäß der geänderten Konfiguration von Befehlen, herzustellen.

Die **Figuren 5A-5D** zeigen Beispiele von Geflechten G, welche mit den Verfahren, Programmen und/oder Vorrichtungen der vorliegenden Erfindung gemäß entsprechender Konfigurationen von Befehlen herstellbar sind. **Figur 5A** zeigt beispielhaft ein offenes Geflecht (Endlosgeflecht), welches einen im Wesentlichen konstanten Durchmesser und Teilabschnitte mit unterschiedlichen Flechtmustern aufweist. Das Geflecht weist sowohl engmaschige als auch breitmaschige Teilabschnitte sowie Übergangsbereich zwischen diesen Teilabschnitten auf. Die Teilabschnitte unterscheiden sich beispielsweise bei Flechtwinkel, Rautenlänge und/oder Rautensegmentlänge. Ein Stent welcher ein Flechtmuster wie in der Figur 5A gezeigt aufweist, ist insbesondere zur Behandlung von bzw. Einsatz bei Aneurysmen geeignet, wobei ein engmaschiger Teilabschnitt an der Stelle des Aneurysmas positioniert wird. **Figur 5B** zeigt ein Geflecht mit Schlaufen an einem Ende des Geflechts. Beim Herstellen eines solchen Geflechts ist insbesondere auf ein möglichst frühes Einstellen der gewünschten Rautengeometrie zu achten. Meist weichen die Formen der jeweils ersten Rauten von denen der folgenden Rauten ab. Bei medizinischen Implantaten, wie z.B. Stents, erzeugen solche oder ähnliche Schlaufen an einem Ende eine Rückstellkraft, sodass der Stent an diesem Ende aufgeweitet wird. **Figur 5C** zeigt ein weiteres beispielhaftes Geflecht, welches auf einem profilierten Flechtdorn erstellt wurde. Bei medizinischen Implantaten kann es vorteilhaft sein, dass die Geometrie/Form des Geflechts an die Form des Gefäßes, in das dieses eingesetzt werden soll, angepasst wird. Insbesondere bei einem profilierten Flechtdorn 4 (z.B. Flechtdorn 4 mit ein oder mehreren Änderungen des Dorndurchmessers) ist es vorteilhaft, dass die vorgegebene bzw. vorgebbare Position (Soll-Kenngröße) einer Überkreuzung Ü des Geflechts G erfassbar und in Relation zu einem oder mehreren (Profil-) Merkmalen des Flechtdorns 4 abgleichbar ist. Ein solcher Vorgang ist insbesondere vorteilhaft, um Kontenpunkte/Überkreuzungen Ü im Bereich einer Änderung des Dornprofils exakt bzw. mit einer möglichst geringen Abweichung zu erzeugen und/oder zu platzieren. Ferner kann eine unerwünschte Abweichung des erzeugten Geflechts G durch Kumulieren bzw. Summieren von innerhalb einer festgelegten Toleranz befindlichen Kenngrößenabweichungen entstehen. Durch Abgleich der Position einer oder mehrerer Überkreuzungen in Relation zu einem oder mehreren Bezugspunkten, welche nicht Bestandteil des Geflechts sind, sind Abweichungen des Geflechts vorteilhafterweise detektierbar und durch eine entsprechende Korrektur reduzierbar bzw. behebbar, insbesondere reduzierbar unter eine vorgegebene Toleranz. **Figur 5D** zeigt ein beispielhaftes Flachgeflecht G (Litze), welches durch die Verfahren und Vorrichtungen der vorliegenden Anmeldung herstellbar ist. Bevorzugt überkreuzt sich bei Flachgeflechten das Flechtmaterial F und kehrt an den Rändern des Flachgeflechts G um. Vorteilhafterweise wird das Flechtmaterial F an den Rändern des Geflechts G zumindest teilweise um bzw. an einen Flechtdorn 4 angelegt, wobei weiter bevorzugt ein Flechtdorn 4 an jeweils einem Rand des Geflechts G vorgesehen ist. Alternativ und/oder zusätzlich kann ein Flachgeflecht ein oder mehrere Stehfäden bzw. Stehdrähte aufweisen, insbesondere an dessen Ränder. Ein Stehfaden bzw. Stehdraht kann identisch zu dem Flechtmaterial F des Geflechts sein oder andere Eigenschaften (z.B. Material, Dicke, Oberflächenbeschaffenheit, ...) aufweisen. Ein Flachgeflecht kann Bereiche mit unterschiedlichen Geometrien und/oder Flechtmustern aufweisen. Bevorzugt ist das Verhältnis zwischen einer räumlichen Ausdehnung in einer ersten Richtung (Dicke - senkrecht zur Bildebene) und in einer zweiten Richtung (Breite - oben-unten in Bildebene) eines Flachgeflechts im Bereich von etwa 1:1,5 bis etwa 1:100, weiter bevorzugt im Bereich von etwa 1:3 bis etwa 1:50, weiter bevorzugt im Bereich von etwa 1:5 bis etwa 1:20 ist, wobei die erste Richtung (Dicke) und die zweite Richtung (Breite) im Wesentlichen orthogonal zueinander und zu der Längsachse des Flechtdorns 4 sind. Die Dicke eines Flachgefechts wird insbesondere durch den Durchmesser des Flechtmaterials F, das Flechtmuster und/oder die geometrischen Eigenschaften eines oder mehrerer etwaiger Flechtdorne bzw. Stehfäden/Stehdrähte bestimmt. Die Breite des Flachgeflechts wird beispielsweise durch den Abstand etwaiger Flechtdorne bzw. Stehfäden/Stehdrähte bestimmt, welcher wiederum beispielsweise durch die Verlagerung des Geflechts und/oder die Variation der Drahtspannung bestimmt und beliebig eingestellt werden kann. Die Länge eines Flachgeflechts (links-rechts in Bildebene) kann beliebig bestimmt werden und insbesondere durch einen stetig laufenden Flechtvorgang beliebig vergrößert werden (Endlosgeflecht).

Weitere (nicht gezeigte) Geflechte können beispielsweise Verdrillungen, Verzweigungen, flach geflochtene Bereiche und Rundflechten beinhalten. Auch Geflechte mit Kombinationen der gezeigten und genannten Formen/Eigenschaften sind mit den Verfahren, Programmen und/oder Vorrichtungen der vorliegenden Erfindung herstellbar. Insbesondere bei Änderungen des Durchmessers bzw. der Form des Flechtdorns können mehrere Iterationen von Änderungs- bzw. Anpassungsvorgängen der Konfiguration von Befehlen notwendig sein, um ein Geflecht mit den gewünschten bzw. vorgegebenen Eigenschaften zu erhalten. Durch die vorliegende Erfindung kann der Aufwand hierfür reduziert werden.

**Figur 6** illustriert Schritte einer bevorzugten Ausführungsform des computergestützten Verfahrens zum Erstellen eines Flechtprogramms gemäß einem Aspekt der Erfindung. Das Verfahren ist dazu geeignet von einer Vorrichtung 1 gemäß einer Ausführungsform der Erfindung durchgeführt zu werden. In einem ersten Schritt wird einer Steuereinheit S eine Konfiguration von Befehlen bereitgestellt, beispielsweise von einem Speichermedium. Die Konfiguration von Befehlen umfasst Befehle, welche ein Verlagern eines oder mehrerer Klöppel K durch eine von der Steuereinheit S gesteuerte Verlagerungseinrichtung 2 (z.B. ein Drehteller und/oder ein Flügelrad) der Vorrichtung 1 bewirkt. Bevorzugt umfasst die Konfiguration von Befehlen ebenfalls Befehle, welche beispielsweise einen Abzug 6 steuert, um das Geflecht zu verlagern, insbesondere durch Verlagern eines mit diesem gekoppelten Flechtdorns 4. Durch die Ausführung der Befehle durch die Steuereinheit S wird in einem zweiten Schritt ein Geflecht G durch Flechten, beispielsweise Umflechten eines Flechtdorns 4, mit von einem oder mehreren Klöppeln K bereitgestelltem Flechtmaterial F erzeugt.

Erfindungsgemäß werden während des Flechtvorgangs in einem dritten Schritt von der Steuereinheit S ein öder mehrere Kenngrößen des entstehenden Geflechts G erfasst und - in einem vierten Schritt - mit zumindest einer entsprechenden bzw. dazugehörigen Soll-Kenngröße abgeglichen. Hierfür erhält die Steuereinheit S von einem oder mehreren gekoppelten Prüfmitteln 18 Informationen bezüglich der zumindest einen erfassten Kenngröße des Geflechts G. Solch ein Prüfmittel 18 ist beispielweise eine Kamera, bevorzugt eine hochauflösende Hochgeschwindigkeitskamera, welche geeignet ist, ein oder mehrere Kenngrößen des Geflechts G zu erfassen und an die Steuereinheit S zu übermitteln. Eine solche Kenngröße ist beispielsweise eine Länge oder Breite einer von Überkreuzungen Ü des Flechtmaterials F gebildeten Raute 20. Die Länge bzw. Breite einer Raute 20 des Geflechts G entspricht dabei bevorzugt der Distanz zwischen sich gegenüberstehenden Überkreuzungen Ü des Flechtmaterials F in axialer Richtung bzw. in radialer Richtung des Geflechts G bzw. des Flechtdorns 4.

Die Steuereinheit S gleicht eine von einem Prüfmittel 18 erfasste Kenngröße mit einer dazugehörigen, vorgegebenen bzw. vorgebbaren Soll-Kenngröße ab. Eine oder mehrere Soll-Kenngrößen werden beispielsweise der Steuereinheit S von einem Datenspeicher bzw. Datenbank D zum Abgleich bereitgestellt bzw. vorgegeben. Ein negatives Abgleichergebnis liegt vor, wenn eine erfasste Kenngröße nicht im Wesentlichen zur dazugehörigen Soll-Kenngröße identisch ist bzw. dieser entspricht, bzw. nicht innerhalb eines festgelegten Toleranzbereichs liegt im Vergleich zur dazugehörigen Soll-Kenngröße.

Bei einem negativen Abgleichergebnis wird in einem fünften Verfahrensschritt der Flechtvorgang durch die Steuereinheit S gestoppt und die Vorrichtung 1 derart gesteuert, dass zumindest ein Bereich des Geflechts G entflochten wird. Dies geschieht beispielsweise durch ein umgekehrtes Verlagern einer oder mehrerer Klöppel K und/oder des Geflechts G bzw. des Flechtdorns 4. Vorteilhafterweise wird lediglich der Abschnitt des Geflechts G entflochten, welcher die abweichende Kenngröße aufweist bzw. dieser entspricht.

In einem sechsten Schritt erfolgt durch die Steuereinheit S eine Änderung zumindest eines Teils der Konfiguration von Befehlen, auf Basis derer das Erzeugen des Geflechts G durchgeführt wurde bzw. wird.

Durch die Änderung der Konfiguration der Befehle soll die abweichende Kenngröße derart geändert werden, dass ein positives Abgleichergebnis erreicht wird, d.h. dass die erfasste Kenngröße im Wesentlichen identisch (bzw. entsprechend) ist bzw. innerhalb eines festgelegten Toleranzbereichs liegt im Vergleich zu der dazugehörigen Soll-Kenngröße. Beispielsweise kann bei einer zu kurzen Rautenlänge die Geschwindigkeit der Verlagerung eines oder mehrerer Klöppel K verlangsamt werden. Alternativ und/oder zusätzlich kann die Geschwindigkeit der Verlagerung des Geflechts G bzw. eines Flechtdorns 4 erhöht werden.

Die Entscheidung, welcher bzw. welche Parameter der Steuerung des Flechtvorgangs geändert wird bzw. werden, wird bevorzugt automatisch von der Steuereinheit S bzw. eines von dieser ausgeführten Algorithmus getroffen.

Besonders bevorzugt erfolgt die Änderung der Parameter bzw. die Änderung der Konfiguration von Befehlen durch: einen Algorithmus auf Basis bzw. unter Berücksichtigung von bestimmten, bereitgestellten Informationen. Solche Informationen werden beispielsweise von der Datenbank D bereitgestellt und/oder umfassen computerlesbare Befehle zur Steuerung einer Flechtmaschine zum Herstellen eines Geflechts. Alternativ und/oder zusätzlich ist der Algorithmus eingerichtet, bereits durchgeführte und/oder hinterlegte Parameteränderungen bzw. Änderungen von Befehlen der Entscheidungsfindung zugrunde zu legen.

In Zusammenhang mit der Selektion der zumindest einen Kenngröße, deren Erfassung, des Abgleiches mit zumindest einer entsprechenden Soll-Kenngröße, mit dem Entflechten und/oder mit dem Ändern der Konfiguration von Befehlen können bevorzugt Algorithmen basierend auf künstlicher Intelligenz bzw. maschinellem Lernen zum Einsatz kommen.

In einem nächsten Schritt wird der Flechtvorgang wieder aufgenommen und das Geflecht gemäß der geänderten Konfiguration von Befehlen erzeugt. Weiter findet während des Flechtvorgangs ein Erfassen einer oder mehrerer Kenngrößen und ein entsprechender Abgleich mit zugehörigen Soll-Kenngrößen statt.

In dem Fall, dass ein positives Abgleichergebnis vorliegt, beispielsweise wenn eine erfasste Kenngröße im Wesentlichen identisch ist bzw. entspricht bzw. innerhalb eines festgelegten Toleranzbereichs liegt im Vergleich zu der dazugehörigen Soll-Kenngröße. In diesem Fall, und falls das Geflecht noch nicht fertig erstellt wurde, wird das Erzeugen des Geflechts G wiederaufgenommen bzw. weitergeführt.

Falls das Geflecht fertiggestellt ist, wird in einem weiteren Schritt ein Flechtprogramm auf Basis der zuletzt geänderten Konfiguration von Befehlen, d.h. gemäß der Konfiguration von Befehlen, bei der keine abweichende Kenngröße erfasst wurde, erstellt. Das Flechtprogramm wird bevorzugt der Datenbank D zugeführt, um in zukünftigen Entscheidungsfindungen von Parameteränderungen berücksichtigt werden zu können.

Die in Figur 6 dargestellten und im Vorgang beschriebenen Schritte eines Verfahrens gemäß eines Aspekts der Erfindung entsprechen einer beispielhaften und besonders bevorzugten Ausführungsform und dienen vordergründig der Veranschaulichung.

### Bezugszeichenliste

- 1: Vorrichtung
- 2: Verlagerungseinrichtung
- 4: Flechtdorn
- 6: Abzug
- 8: Flechtring
- 10: Flechtauge
- 12: Luftbereich
- 14: Ablagebereich
- 16: Fixbereich
- 18: Prüfmittel
- 20: Raute
- 22: Rautensegment
- 24: Rautenlänge
- 26: Rautenbreite
- 28: Flechtwinkel
- 30: Geflecht Teilabschnitt (Rautendichte)
- 32: Geflechtdurchmesser
- 33: Flechtdornlängsachse
- 34: Verbindungslinie Überkreuzungen/Knotenpunkte

- D: Datenbank
- F: Flechtmaterial
- G: Geflecht
- K: Klöppel
- L1, L2: Laufbahn
- S: Steuereinheit
- Ü: Überkreuzung/Knotenpunkt
- W: Weiche

## Patentansprüche

1. Computer-gestütztes Verfahren zum Erstellen eines Flechtprogramms, wobei das Flechtprogramm maschinenlesbare Befehle umfasst, die beim Ausführen des Flechtprogramms eine Flechtmaschine veranlassen, ein Geflecht (G), insbesondere ein zumindest teilweise geflochtenes Implantat, herzustellen, wobei das Verfahren umfasst:
a) Erzeugen eines Geflechts (G) durch die Flechtmaschine, insbesondere durch Umflechten zumindest eines Flechtdorns (4), mit einem Flechtmaterial (F) gemäß einer vorgegebenen Konfiguration von Befehlen;
b) automatisiertes Erfassen zumindest einer Kenngröße des Geflechts (G);
c) automatisiertes Abgleichen der erfassten Kenngröße des Geflechts (G) mit zumindest einer zugehörigen Soll-Kenngröße;
sofern die erfasste Kenngröße der zugehörigen Soll-Kenngröße nicht entspricht:
c1-a) Entflechten zumindest eines Bereichs des Geflechts (G) durch die Flechtmaschine; und
c1-b) automatisiertes Ändern zumindest eines Teils der Konfiguration von Befehlen zum Erzeugen des Geflechts (G) sowie Wiederholen der Schritte a) bis c) auf der Grundlage der geänderten Konfiguration von Befehlen;
sofern die erfasste Kenngröße der zugehörigen Soll-Kenngröße entspricht:
c2) Erstellen eines Flechtprogramms gemäß der Konfiguration von Befehlen bei der die erfasste Kenngröße der zugehörigen Soll-Kenngröße entspricht.

2. Verfahren nach Anspruch 1, wobei das Ändern zumindest eines Teils der Konfiguration von Befehlen abhängig ist von:
Art der abweichenden Kenngröße;
Ausmaß der Abweichung der Kenngröße;
Anzahl der Klöppel;
Art des Flechtmaterials (F);
Dicke des Flechtmaterials (F);
Oberflächenbeschaffenheit des Flechtmaterials (F);
Oberflächenbeschaffenheit eines Flechtdorns (4); und/oder
Geometrie eines Flechtdorns (4).

3. Verfahren nach Anspruch 1 oder 2, wobei das Ändern der Konfiguration von Befehlen durch einen lernfähigen, computerimplementierten Algorithmus beeinflusst wird.

4. Verfahren nach Anspruch 3, wobei der lernfähige, computerimplementierte Algorithmus die Konfiguration von Befehlen ändert auf Basis:
einer vorgegebenen Konfiguration von Befehlen;
einer zuvor erfassten Kenngrößenabweichung;
einer durchgeführten Konfigurationsänderung; und/oder
einer detektierten Auswirkung einer bestimmten Konfigurationsänderung.

5. Verfahren nach einem der vorhergehenden Ansprüche, wobei die Konfiguration von Befehlen zum Erzeugen des Geflechts ein oder mehrere der folgenden Parameter umfasst:
Verlagerung zumindest eines Klöppels (K);
Verlagerung des Geflechts (G);
Verlagerung eines Flechtdorns (4);
Position eines Flechtrings (8);
Position eines Flechtauges (10);
Spannung des Flechtmaterials (F).

6. Verfahren nach einem der vorhergehenden Ansprüche, wobei eine Kenngröße des Geflechts (G) beinhaltet:
Position zumindest einer Überkreuzung des Flechtmaterials (F), insbesondere in Relation zu zumindest einem Merkmal eines Flechtdorns (4);
Durchmesser des Flechtmaterials (F);
Länge einer Raute (20);
Breite einer Raute (20);
Länge eines Rautensegments (22); und/oder
Flechtwinkel einer Raute (20).

7. Verfahren nach einem der vorhergehenden Ansprüche, wobei das Erfassen einer Kenngröße des Geflechts (G) in zumindest einem bestimmten Bereich des Geflechts (G), bevorzugt in Relation zu einem Flechtdorn (4), erfolgt, insbesondere in einem Luftbereich (12), in einem Ablagebereich (14) und/oder in einem Fixbereich (16) des Geflechts.

8. Verfahren nach einem der vorhergehenden Ansprüche, wobei das Entflechten ein im Wesentlichen umgekehrtes Verlagern zumindest eines Klöppels (K) und/oder des Geflechts (G) und/oder eines Flechtdorns (4) umfasst.

9. Verfahren nach einem der vorhergehenden Ansprüche, wobei das Entflechten zumindest bis zu dem Bereich des Geflechts (G) erfolgt, welcher die von der zugehörigen Soll-Kenngröße abweichende Kenngröße aufweist.

10. Computerprogramm, umfassend Befehle, die beim Ausführen des Programms durch eine Vorrichtung diese veranlassen, das Verfahren zum Erstellen eines Flechtprogramms gemäß einem der Ansprüche 1-9 auszuführen.

11. Vorrichtung (1) zum Erstellen eines Flechtprogramms, wobei das Flechtprogramm maschinenlesbare Befehle umfasst, die beim Ausführen des Flechtprogramms eine Flechtmaschine (1) veranlassen, ein Geflecht (G), insbesondere ein zumindest teilweise geflochtenes Implantat, herzustellen, wobei die Vorrichtung (1) zur Durchführung des Verfahrens gemäß Ansprüchen 1-9 geeignet ist und umfasst:
zumindest einen Klöppel (K) zum Führen eines Flechtmaterials (F), wobei der zumindest eine Klöppel (K) verlagerbar ist, um sich entlang von Laufbahnen, insbesondere von beliebig vorgebbaren Laufbahnen, zu bewegen;
eine Verlagerungseinrichtung (2) zum Verlagern des zumindest einen Klöppels (K) entlang einer Laufbahn;
einen Abzug (6) zum Verlagern des Geflechts (G), insbesondere zum Aufnehmen und Verlagern zumindest eines Flechtdorns (4);
eine Steuereinheit zum Steuern des Verlagerns des zumindest einen Klöppels (K) und des Geflechts (G), insbesondere des zumindest einen Flechtdorns (4), um so ein Geflecht (G) durch Flechten, insbesondere durch Umflechten des zumindest einen Flechtdorns (4), mit dem Flechtmaterial (F) zu erzeugen; und
ein Prüfmittel (18) zum Erfassen zumindest einer Kenngröße eines von der Vorrichtung (1) erzeugten Geflechts (G);
wobei die Steuereinheit ferner eingerichtet ist zum:
a) Erzeugen eines Geflechts (G) durch die Flechtmaschine, insbesondere durch Umflechten zumindest eines Flechtdorns (4), mit einem Flechtmaterial (F) gemäß einer vorgegebenen Konfiguration von Befehlen;
b) automatisiertes Erfassen zumindest einer Kenngröße des Geflechts (G);
c) automatisiertes Abgleichen der zumindest einen erfassten Kenngröße des Geflechts (G) mit zumindest einer zugehörigen Soll-Kenngröße;
sofern die erfasste Kenngröße der zugehörigen Soll-Kenngröße nicht entspricht:
c1-a) Entflechten zumindest eines Bereichs des Geflechts (G) durch die Flechtmaschine; und
c1-b) automatisiertes Ändern zumindest eines Teils der Konfiguration von Befehlen zum Erzeugen Geflechts (G) sowie Wiederholen der Schritte a) bis c) auf der Grundlage der geänderten Konfiguration von Befehlen;
sofern die erfasste Kenngröße der zugehörigen Soll-Kenngröße entspricht:
c2) Erstellen eines Flechtprogramms gemäß der Konfiguration von Befehlen bei der die erfasste Kenngröße der zugehörigen Soll-Kenngröße entspricht.

## Claims

1. Computer-aided process for creating a braiding program,
wherein the braiding program comprises machine-readable commands, which, during execution of the braiding program, induce a braiding machine to produce a braided structure (G), in particular, an at least partially braided implant, wherein the process comprises:
a) generating a braided structure (G) by the braiding machine, in particular by braid-wrapping at least one braid mandrel (4) using a braiding material (F) according to a specified configuration of commands;
b) automatically capturing at least one parameter of the braided structure (G);
c) automatically reconciling the captured parameter of the braided structure (G) with at least one associated target parameter;
provided the captured parameter does not correspond to the associated target parameter:
c1-a) unbraiding at least one region of the braided structure (G) by the braiding machine; and
c1-b) automatically changing at least part of the configuration of commands for generating the braided structure (G)
as well as repeating steps a) to c) based on the changed configuration of commands;
provided the captured parameter corresponds to the associated target parameter;
c2) creating a braiding program according to the configuration of commands, in which the captured parameter corresponds to the associated target parameter.

2. Process according to claim 1, wherein the change of at least part of the configuration of commands is dependent on:
type of deviating parameter;
extent of the deviation of the parameter;
number of bobbins;
type of braiding material (F);
thickness of braiding material (F);
surface quality of the braiding material (F);
surface quality of a braid mandrel (4); and/or
geometry of a braid mandrel (4).

3. Process according to claim 1 or 2, wherein the change of the configuration of commands is influenced by a teachable computer-implemented algorithm.

4. Process according to claim 3, wherein the teachable, computer-implemented algorithm changes the configuration of commands based on:
a specified configuration of commands;
a previously captured parameter deviation;
an implemented configuration change; and/or
a detected effect of a specific configuration change.

5. Process according to any one of the preceding claims, wherein the configuration of commands for generating the braided structure comprises one or more of the following parameters:
relocation of at least one bobbin (K);
relocation of the braided structure (G);
relocation of a braid mandrel (4);
position of a braid ring (8);
position of a braid eye (10);
tension of the braiding material (F).

6. Process according to any one of the preceding claims, wherein a parameter of the braided structure (G) comprises:
position of at least one crossover of the braiding material (F), in particular in relation to at least one feature of a braid mandrel (4);
diameter of the braiding material (F);
length of a rhombus (20);
width of a rhombus (20);
length of a rhombus segment (22); and/or
braid angle of a rhombus (20).

7. Process according to any one of the preceding claims, wherein the capture of a parameter of the braided structure (G) takes place in at least one specific region of the braided structure (G), preferably in relation to a braid mandrel (4), in particular in an aerial region (12), in a dropping region (14) and/or in a fixing region (16) of the braided structure.

8. Process according to any one of the preceding claims, wherein the unbraiding comprises a substantially reversed relocation of at least one bobbin (K) and/or of the braided structure (G) and/or of a braid mandrel (4).

9. Process according to any one of the preceding claims, wherein the unbraiding takes place at least up to the region of the braided structure (G) which has the parameter that deviates from the associated target parameter.

10. Computer program comprising commands which, during execution of the program by a device, induce the latter to execute the process for creating a braiding program according to any one of claims 1-9.

11. Device (1) for creating a braiding program, wherein the braiding program comprises machine-readable commands, which, during execution of the braiding program, induce a braiding machine (1) to produce a braided structure (G), in particular an at least partially braided implant,
wherein the device (1) is suitable for carrying out the process according to any one of claims 1-9 and comprises:
at least one bobbin (K) for guiding a braiding material (F), wherein the at least one bobbin (K) is relocatable to move along paths, in particular randomly specifiable paths;
a relocation device (2) for relocating the at least one bobbin (K) along a path;
a puller (6) for relocating the braided structure (G), in particular for picking up and relocating at least one braid mandrel (4);
a control unit for controlling the relocation of the at least one bobbin (K) and/or of the braided structure (G), in particular of the at least one braid mandrel (4), in order to generate in this manner a braided structure (G) by braiding, in particular, by braid-wrapping at least one braid mandrel (4) using the braiding material (F); and
a testing means (18) for capturing at least one parameter of a braided structure (G) generated by the device (1);
wherein the control unit is furthermore configured for:
a) generating a braided structure (G) by the braiding machine, in particular by braid-wrapping at least one braid mandrel (4) using a braiding material (F) according to a specified configuration of commands;
b) automatically capturing at least one parameter of the braided structure (G);
c) automatically reconciling the at least one captured parameter of the braided structure (G) with at least one associated target parameter;
provided the captured parameter does not correspond to the associated target parameter:
c1-a) unbraiding at least one region of the braided structure (G) by the braiding machine; and
c1-b) automatically changing at least part of the configuration of commands for generating the braided structure (G) as well as repeating steps a) to c) based on the changed configuration of commands;
provided the captured parameter corresponds to the associated target parameter:
c2) creating a braiding program according to the configuration of commands, in which the captured parameter corresponds to the associated target parameter.

## Revendications

1. Procédé assisté par ordinateur pour créer un programme de tressage,
dans lequel le programme de tressage comprend des instructions lisibles par ordinateur, qui amènent, lors de l'exécution du programme de tressage, une machine de tressage à fabriquer un article tressé (G), en particulier un implant au moins en partie tressé, dans lequel le procédé comprend :
a) la production d'un article tressé (G) par la machine de tressage, en particulier en tressant un matériau de tressage (F) au moins autour d'un mandrin de tressage (4) selon une configuration prédéfinie d'instructions ;
b) la détection automatisée d'au moins une grandeur caractéristique de l'article tressé (G) ;
c) la comparaison automatisée de la grandeur caractéristique détectée de l'article tressé (G) à au moins une grandeur caractéristique de consigne associée ;
dans la mesure où la grandeur caractéristique détectée ne correspond pas à la grandeur caractéristique de consigne associée :
c1-a) le démêlage d'au moins une zone de l'article tressé (G) par la machine de tressage ; et
c1-b) la modification automatisée d'au moins une partie de la configuration d'instructions pour produire l'article tressé (G)
ainsi que la répétition des étapes c) à c) sur la base de la configuration modifiée d'instructions ;
dans la mesure où la grandeur caractéristique détectée correspond à la grandeur caractéristique de consigne associée :
c2) la création d'un programme de tressage selon la configuration d'instructions, où la grandeur caractéristique détectée correspond à la grandeur caractéristique de consigne associée.

2. Procédé selon la revendication 1, dans lequel la modification d'au moins une partie de la configuration d'instructions dépend :
du type de la grandeur caractéristique qui diverge ;
de l'ampleur de la divergence de la grandeur caractéristique ;
du nombre des fuseaux ;
du type du matériau de tressage (F) ;
de l'épaisseur du matériau de tressage (F) ;
de la nature de la surface du matériau de tressage (F) ;
de la nature de la surface d'un mandrin de tressage (4) ; et/ou
de la géométrie d'un mandrin de tressage (4).

3. Procédé selon la revendication 1 ou 2, dans lequel la modification de la configuration d'instructions est influencée par un algorithme d'apprentissage mis en oeuvre par ordinateur.

4. Procédé selon la revendication 3, dans lequel l'algorithme d'apprentissage mis en oeuvre par ordinateur modifie la configuration d'instructions sur la base :
d'une configuration prédéfinie d'instructions ;
d'une divergence de grandeur caractéristique détectée au préalable ;
d'une modification de configuration effectuée ; et/ou
d'un impact détecté d'une modification de configuration donnée.

5. Procédé selon l'une quelconque des revendications précédentes, dans lequel la configuration d'instructions comprend pour produire l'article tressé un ou plusieurs des paramètres suivants :
le déplacement d'au moins un fuseau (K) ;
le déplacement de l'article tressé (G) ;
le déplacement d'un mandrin de tressage (4) ;
la position d'un anneau de tressage (8) ;
la position d'un oeil de tressage (10) ;
la tension du matériau de tressage (F).

6. Procédé selon l'une quelconque des revendications précédentes, dans lequel une grandeur caractéristique de l'article tressé (G) renferme :
la position d'au moins un croisement du matériau de tressage (F), en particulier par rapport à au moins une caractéristique d'un mandrin de tressage (4) ;
le diamètre du matériau de tressage (F) ;
la longueur d'un losange (20) ;
la largeur d'un losange (20) ;
la longueur d'un segment de losange (22) ; et/ou
l'angle de tressage d'un losange (20).

7. Procédé selon l'une quelconque des revendications précédentes, dans lequel la détection d'une grandeur caractéristique de l'article tressé (G) a lieu dans au moins une zone donnée de l'article tressé (G), de manière préférée par rapport à un mandrin de tressage (4), en particulier dans une zone d'air (12), dans une zone de pose (14) et/ou dans une zone fixe (16) de l'article tressé.

8. Procédé selon l'une quelconque des revendications précédentes, dans lequel le démêlage comprend un déplacement sensiblement inversé d'au moins un fuseau (K) et/ou de l'article tressé (G) et/ou d'un mandrin de tressage (4).

9. Procédé selon l'une quelconque des revendications précédentes, dans lequel le démêlage a lieu au moins jusqu'à une zone de l'article tressé (G), laquelle présente la grandeur caractéristique qui diverge de la grandeur caractéristique de consigne associée.

10. Programme informatique comprenant des instructions, qui amènent, lors de l'exécution du programme par un dispositif, celui-ci à exécuter le procédé pour créer un programme de tressage selon l'une quelconque des revendications 1 - 9.

11. Dispositif (1) pour créer un programme de tressage, dans lequel le programme de tressage comprend des instructions lisibles par machine, qui amènent, lors de l'exécution du programme de tressage, une machine de tressage (1) à fabriquer un article tressé (G), en particulier un implant au moins en partie tressé,
dans lequel le dispositif (1) est adapté pour effectuer le procédé selon les revendications 1 - 9 et comprend :
au moins un fuseau (K) destiné à guider un matériau de tressage (F), dans lequel l'au moins un fuseau (K) peut être déplacé pour se déplacer le long de voies de roulement, en particulier de voies de roulement pouvant être prédéfinies de manière quelconque ;
un système de déplacement (2) destiné à déplacer l'au moins un fuseau (K) le long de la voie de roulement ;
une levée (6) destinée à déplacer l'article tressé (G), en particulier destinée à recevoir et à déplacer au moins un mandrin de tressage (4) ;
une unité de commande destinée à commander le déplacement de l'au moins un fuseau (K) et de l'article tressé (G), en particulier de l'au moins un mandrin de tressage (4) pour produire ainsi un article tressé (G) par tressage, en particulier par tressage du matériau de tressage (F) autour de l'au moins un mandrin de tressage (4) ; et
un moyen de vérification (18) destiné à détecter au moins une grandeur caractéristique d'un article tressé (G) produit par le dispositif (1),
dans lequel l'unité de commande est mise au point en outre pour :
a) produire un article tressé (G) par la machine de tressage, en particulier par le tressage d'un matériau de tressage (F) autour d'au moins un mandrin de tressage (4) selon une configuration prédéfinie d'instructions ;
b) détecter de manière automatisée au moins une grandeur caractéristique de l'article tressé (G) ;
c) comparer de manière automatisée l'au moins une grandeur caractéristique détectée de l'article tressé (G) à au moins une grandeur caractéristique de consigne associée ;
dans la mesure où la grandeur caractéristique détectée ne correspond pas à la grandeur caractéristique de consigne associée ;
c1-a) démêler au moins une zone de l'article tressé (G) par la machine de tressage ; et
c1-b) modifier de manière automatisée au moins une partie de la configuration d'instructions pour produire l'article tressé (G) ainsi que répéter les étapes a) à c) sur la base de la configuration modifiée d'instructions ;
dans la mesure où la grandeur caractéristique détectée correspond à la grandeur caractéristique associée :
c2) créer un programme de tressage selon la configuration d'instructions, où la grandeur caractéristique détectée correspond à la grandeur caractéristique de consigne associée.
